# EUROPÄISCHE PATENTSCHRIFT

(11) **EP 1 292 569 B1**
(45) Veröffentlichungstag und Bekanntmachung des Hinweises auf die Patenterteilung: **30.11.2005**
(21) Anmeldenummer: 01953979.0
(22) Anmeldetag: 13.06.2001
(51) Int. Cl.: C07C 253/34, C07C 255/24, C07C 209/48

(54) **VERFAHREN ZUR ABTRENNUNG VON 6-AMINOCAPRONITRIL AUS GEMISCHEN, DIE 6-AMINOCAPRONITRIL, ADIPODINITRIL UND HEXAMETHYLENDIAMIN ENTHALTEN**
METHOD FOR REMOVING 6-AMINOCAPRONITRILE FROM MIXTURES THAT CONTAIN 6-AMINOCAPRONITRILE, ADIPODINITRILE AND HEXAMETHYLENEDIAMINE
PROCEDE DE SEPARATION DE 6-AMINOCAPRONITRILE A PARTIR DE MELANGES DE 6-AMIOCAPRONITRILE, D'ADIPODINITRILE ET D'HEXAMETHYLENEDIAMINE

(30) Priorität: 19.06.2000 DE 10029187
(43) Veröffentlichungstag der Anmeldung: 19.03.2003
(73) Patentinhaber: BASF Aktiengesellschaft, 67056 Ludwigshafen (DE)
(72) Erfinder: LUYKEN, Hermann, 67069 Ludwigshafen (DE); OHLBACH, Frank, Dr., 40219 Düsseldorf (DE); FISCHER, Rolf-Hartmuth, 69121 Heidelberg (DE); MELDER, Johann-Peter, 67459 Böhl-Iggelheim (DE); BASSLER, Peter, 68519 Viernheim (DE); ANSMANN, Andreas, 69168 Wiesloch (DE); ACHHAMMER, Günther, 68309 Mannheim (DE)
(86) Internationale Anmeldenummer: PCT/EP2001/006688
(87) Internationale Veröffentlichungsnummer: WO 2001/098261

(56) Entgegenhaltungen:
- WO-A-96/20931
- WO-A-97/23454

## Beschreibung

Die vorliegende Erfindung betrifft ein Verfahren zur destillativen Abtrennung von 6-Aminocapronitril aus Gemischen, die 6-Aminocapronitril, Adipodinitril und Hexamethylendiamin enthalten, wobei man
a) Hexamethylendiamin aus dem Gemisch abtrennt, unter Erhalt einer Mischung (I), die einen Hexamethylendiamin-Gehalt von weniger als 1 Gew.-% enthält
b) 6-Aminocapronitril ganz oder teilweise aus Mischung (I) abtrennt, unter Erhalt einer Mischung (II), deren Gehalt an Stoffen, die unter Destillationsbedingungen höher als 6-Aminocapronitril sieden und nicht durch Dimerisierungsreaktionen bei thermischer Belastung von 6-Aminocapronitril entstehen können, kleiner als 1 Gew.-% ist und
c) aus Mischung (II) ganz oder teilweise vorhandenes Hexamethylendiamin abtrennt unter Erhalt einer Mischung (IV), deren Hexamethylendiamin-Gehalt höher als in Mischung (II) ist und einer Mischung (V), deren Hexamethylendiamin-Gehalt geringer als in Mischung (II) ist.

6-Aminocapronitril kann bekanntermaßen zur Herstellung von Caprolactam oder von Polyamiden eingesetzt werden. Für solche Zwecke muß das 6-Aminocapronitril eine hohe Reinheit aufweisen.

Die Herstellung von 6-Aminocapronitril erfolgt üblicherweise durch partielle, katalytische Hydrierung von Adipodinitril, welches durch doppelte Hydrocyanierung von Butadien in Gegenwart von Katalysatoren erhalten werden kann. Dabei erhält man in der Regel ein Gemisch, das 6-Aminocapronitril, nicht umgesetztes Adipodinitril und Hexamethylendiamin, sowie gegebenenfalls Lösungsmittel, Leichtsieder bezüglich Hexamethylendiamin, Hochsieder bezüglich Adipodinitril und andere Nebenprodukte enthält.

Es sind zahlreiche Verfahren zur Abtrennung von 6-Aminocapronitril aus solchen Gemischen unter Erhalt eines 6-Aminocapronitrils in der genannten hohen Reinheit bekannt, beispielsweise aus WO 96/20931 und WO97/23454.

Nachteilig bei diesen Verfahren ist der hohe Energieaufwand, der zur Gewinnung von 6-Aminocapronitril ausreichender Reinheit aus dem Gemisch erforderlich ist.

Der vorliegenden Erfindung liegt die Aufgabe zugrunde, ein Verfahren zur Verfügung zu stellen, das die Gewinnung von 6-Aminocapronitril ausreichender Reinheit aus Gemischen, die 6-Aminocapronitril, Adipodinitril, Hexamethylendiamin und gegebenenfalls Lösungsmittel und andere Nebenkomponenten enthalten, auf technisch einfache und wirtschaftliche Weise ermöglicht unter Vermeidung der genannten Nachteile.

Demgemäß wurde das eingangs definierte Verfahren gefunden.

Verfahren zur Herstellung von Gemischen, die 6-Aminocapronitril, Adipodinitril, Hexamethylendiamin und gegebenenfalls Lösungsmittel, Leichtsieder bezüglich Hexamethylendiamin, Hochsieder bezüglich Adipodinitril und andere Nebenprodukte enthalten, sind an sich bekannt.

So können Gemische, die 6-Aminocapronitril, Adipodinitril und Hexamethylendiamin enthalten, durch katalytische partielle Hydrierung von Adipodinitril erhalten werden.

Bezogen auf die Summe aus 6-Aminocapronitril, Adipodinitril und Hexamethylendiamin enthalten solche Gemische üblicherweise von 5 bis 90 Gew.-%, vorzugsweise 10 bis 80 Gew.-%, insbesondere 20 bis 70 Gew.-% 6-Aminocapronitril, von 5 bis 90 Gew.-%, vorzugsweise 10 bis 80 Gew.-%, insbesondere 20 bis 70 Gew.-% Hexamethylendiamin, Rest nicht umgesetztes Adipodinitril.

Der zur Hydrierung eingesetzte Katalysator ist nach bisherigen Beobachtungen hinsichtlich des erfindungsgemäßen Verfahrens unkritisch. Das Gemisch kann von dem Katalysator nach der Hydrierung in an sich bekannter Weise, beispielsweise durch Filtration, vorzugsweise im Falle einer Suspensionshydrierung, oder durch Entfernen des Gemisches aus dem Reaktionsgefäß unter Verbleib des Katalysators im Reaktionsgefäß, vorzugsweise im Falle einer Festbetthydrierung, erfolgen.

Vorteilhaft kann man die Hydrierung in Gegenwart eines Lösungsmittels durchführen. Es kommen organische Lösungsmittel, wie Alkohole, vorzugsweise Alkanole, insbesondere C₁-C₄-Alkanole, wie Methanol, Ethanol, n-Propanol, i-Propanol, n-Butanol, i-Butanol, s-Butanol, Ester, vorzugsweise solche einer Alkancarbonsäure, insbesondere einer C₁-C₄-Alkancarbonsäure, wie Ameisensäure, Essigsäure, Propionsäure, Buttersäure, mit einem Alkanol, insbesondere einem C₁-C₄-Alkanol, wie Methanol, Ethanol, n-Propanol, i-Propanol, n-Butanol, i-Butanol, s-Butanol, Ether, wie lineare oder cyclische Ether, beispielsweise Dimethylether, Diethylether, Methyl-t-butyl-ether, Tetrahydrofuran, Kohlenwasserstoffe, wie aliphatische oder aromatische Kohlenwasserstoffe, beispielsweise Benzol, Toluol, Ethylbenzol, o-Xylol, m-Xylol, p-Xylol, Amine, wie primäre, sekundäre oder tertiäre Amine, Lactone, wie Butyrolacton, Lactame, wie Caprolactam, Pyrrolidon, N-Methyl-pyrrolidon, Amide oder anorganische Lösungsmittel, wie Ammoniak, oder deren Gemische in Betracht.

Bevorzugt sind Alkohole, insbesondere Methanol und Ethanol, aromatische Kohlenwasserstoffe, insbesondere Toluol, und Ammoniak oder deren Gemische.

Führt man die Hydrierung in Gegenwart eines Lösungsmittels durch, so enthält das Reaktionsgemisch anschließend dieses Lösungsmittel. Vor dem erfindungsgemäßen Verfahren kann das Lösungsmittel vorteilhaft in an sich bekannter Weise, beispielsweise durch Destillation oder Rektifikation, von dem Reaktionsgemisch abgetrennt werden.

Diese Abtrennung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, mit oder ohne Seitenabzug.

Bei der Hydrierung können Nebenprodukte entstehen, beispielsweise Leichtsieder bezüglich Hexamethylendiamin und/oder Stoffe, die höher als Adipodinitril sieden.

Unter Leichtsieder bezüglich Hexamethylendiamin werden im Sinne der vorliegenden Erfindung Verbindungen verstanden, die unter den entsprechenden Destillationsbedingungen einen Siedepunkt unterhalb dessen von Hexamethylendiamin aufweisen.

Als Leichtsieder bezüglich Hexamethylendiamin kommen beispielsweise Hexamethylenimin, Hexylamin, Aminomethylcyclopentylamin, Diaminocyclohexan in Betracht. Solche Verbindungen liegen in den bei der Hydrierung erhaltenen Gemischen in Summe in Mengen von bis zu 10 Gew.-%, vorzugsweise bis 5 Gew.-%, bezogen auf die Summe aus 6-Aminocapronitril, Adipodinitril und Hexamethylendiamin, vor.

Vor dem erfindungsgemäßen Verfahren können Leichtsieder bezüglich Hexamethylendiamin vorteilhaft in an sich bekannter Weise, beispielsweise durch Destillation der Rektifikation von dem Reaktionsgemisch abgetrennt werden.

Diese Abtrennung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, mit oder ohne Seitenabzug.

Gegebenenfalls eingesetztes Lösungsmittel und gegebenenfalls vorhandene Leichtsieder hinsichtlich Hexamethylendiamin können gleichzeitig in Schritt a oder vorher abgetrennt werden. Vorzugsweise trennt man zunächst gegebenenfalls eingesetztes Lösungsmittel (Schritt a0) und dann gegebenenfalls vorhandene Leichtsieder hinsichtlich Hexamethylendiamin (Schritt a1) ab.

Schritt a1 sollte vorteilhaft bei einem Druck unterhalb atmosphärischen Umgebungsdrucks, vorzugsweise bei einem Druck von weniger als 500 mbar absolut durchgeführt werden.

Erfindungsgemäß trennt man in Schritt a Hexamethylendiamin aus dem Gemisch ab unter Erhalt einer Mischung (I), die einen Hexamethylendiamin-Gehalt von weniger als 5 Gew.-%, vorzugsweise weniger als 1 Gew.-% bezogen auf Mischung (I) aufweist.

Diese Abtrennung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, mit oder ohne, vorzugsweise mit Seitenabzug.

Schritt a sollte vorteilhaft bei einem Druck unterhalb atmosphärischen Umgebungsdrucks, vorzugsweise bei einem Druck von weniger als 500 mbar absolut durchgeführt werden.

In einer bevorzugten Ausführungsform kann man Hexamethylendiamin und bezüglich Hexamethylendiamin leichter siedende Komponenten gemeinsam abtrennen, also Schritt a1 und a gemeinsam durchführen.

Dabei kann Hexamethylendiamin vorteilhaft an einem Seitenabzug gewonnen werden.

In einer besonders bevorzugten Ausführungsform verwendet man eine Kolonne mit einem Trennblech im Bereich zwischen Zulauf und Seitenabzug.

Erfindungsgemäß trennt man in Schritt b 6-Aminocapronitril ganz oder teilweise aus Mischung (I) ab unter Erhalt einer Mischung (II), deren Gehalt an Stoffen, die unter Destillationsbedingungen höher als 6-Aminocapronitril sieden und nicht durch Dimerisierungsreaktionen bei thermischer Belastung von 6-Aminocapronitril entstehen können, kleiner als 2 Gew.-%, vorzugsweise kleiner als 0,1 Gew.-%, ist.

Diese Abtrennung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, mit oder ohne, vorzugsweise mit Seitenabzug.

Schritt b sollte vorteilhaft bei einem Druck unterhalb atmosphärischen Umgebungsdrucks, vorzugsweise bei einem Druck von weniger als 500 mbar absolut durchgeführt werden.

Erfindungsgemäß trennt man in Schritt c aus Mischung (II) ganz oder teilweise vorhandenes Hexamethylendiamin ab unter Erhalt einer Mischung (IV), deren Hexamethylendiamin-Gehalt höher als in Mischung (II) ist und einer Mischung (V), deren Hexamethylendiamin-Gehalt geringer als in Mischung (II) ist.

Diese Abtrennung kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen, mit oder ohne Seitenabzug.

Schritt c sollte vorteilhaft bei einem Druck unterhalb atmosphärischen Umgebungsdrucks, vorzugsweise bei einem Druck von weniger als 500 mbar absolut durchgeführt werden.

In einer bevorzugten Ausführungsform sollte der Druck in Schritt a geringer sein als der Druck in Schritt c.

In einer weiteren bevorzugten Ausführungsform kann die in Schritt c erhaltene Mischung (IV) in Schritt a zurückgeführt werden. Dabei ist Mischung (IV) vorzugsweise dampfförmig.

In einer weiteren Ausführungsform von Schritt b im Sinne der vorliegenden Erfindung kann man einen höheren Gehalt an Hochsiedern bezüglich 6-Aminocapronitril zulassen unter Erhalt einer Mischung (IIa), bezüglich 6-Aminocapronitril schwerer siedende Komponenten aus Mischung (IIa) abtrennen unter Erhalt einer Mischung (VI), deren 6-Aminocapronitril-Gehalt höher als in Mischung (II) ist und einer Mischung (VII), deren 6-Aminocapronitril-Gehalt niedriger als in Mischung (II) ist.

In einer weiteren bevorzugten Ausführungsform kann man Mischung (VII) in Schritt b zurückführen.

Der Gehalt an Hexamethylendiamin in Mischung (V) sollte vorteilhaft höchstens 5000 ppm, vorzugsweise 0 bis 1000 ppm, insbesondere 0 bis 200 ppm, bezogen auf das Gewicht von Mischung (V) betragen. Die Summe der Komponenten von Mischung (V) außer 6-Aminocapronitril sollte vorteilhaft höchstens 5000 ppm, vorzugsweise 0 bis 500 ppm, insbesondere 0 bis 200 ppm, bezogen auf das Gewicht von Mischung (V) betragen.

Der Gesamtenergieverbrauch des erfindungsmäßigen Verfahrens, berechnet als die Summe der Energieverbräuche in den Schritten a, b und c, sowie gegebenenfalls a0 und a1, ist niedriger als der Energieverbrauch, der benötigt wird, wenn man die Abtrennung in nur zwei Schritten a und c, sowie gegebenenfalls a0 und a1, durchführt und den gleichen Hexamethylendiamin-Gehalt im abgetrennten 6-Aminocapronitril erreicht.

### Beispiel

In einer Destillationskolonne K1 wurden 200 kg/h Hydriergemisch, enthaltend 29 % Hexamethylendiamin, 42 % 6-Aminocapronitril, 27 % Adipodinitril, 0.5 % Leichtsieder und 1.5 % Hochsieder so abgetrennt, dass 1.5 kg/h über Kopf, enthaltend im wesentlichen Leichtsieder, abgezogen wurden.

An einem Seitenabzug wurden 58 kg/h Hexamethylendiamin mit einem 6-Aminocapronitril-Gehalt von 110 ppm abgezogen. Über Sumpf der Kolonne K1 wurden 156.5 kg/h mit einem Hexamethylendiamin-Gehalt von 0.2 % abgezogen. Die Kolonne K1 wurde mit 90 kg/h Dampf betrieben. In einer zweiten Destillationskolonne K2 wurde der Sumpfabzug aus Kolonne K1 so destilliert, dass 116.5 kg/h 6-Aminocapronitril mit einem Hexamethylendiamin-Gehalt von 0.3 % über Kopf abgezogen. Über Sumpf wurden 58 kg/h mit einem 6-Aminocapronitril-Gehalt von 2 % abgezogen. Das Kopfprodukt von Kolonne K2 wurde in einer dritten Kolonne K3 so destilliert, dass über Kopf 17 kg/h dampfförmig abgezogen und in Kolonne K1 zurückgeführt wurden. Die Kolonne K2 wurde mit 30 kg/h Dampf betrieben. An einem Seitenabzug von Kolonne K3 wurden 99.5 kg/h 6-Aminocapronitril mit einem Hexamethylendiamin-Gehalt von 45 ppm abgezogen. Die Kolonne K3 wurde mit 5 kg/h Dampf betrieben. Der Dampfverbrauch der Kolonnen K1 + K3 betrug 95 kg/h.

### Vergleichsbeispiel

In der Destillationskolonne K1 gemäß Beispiel wurden 200 kg/h Hydriergemisch, enthaltend 29 % Hexamethylendiamin, 42 % 6-Aminocapronitril, 27 % Adipodinitril, 0.5 % Leichtsieder und 1.5 % Hochsieder so abgetrennt, dass 1.5 kg/h über Kopf, enthaltend im wesentlichen Leichtsieder, abgezogen wurden.

An einem Seitenabzug wurden 58 kg/h Hexamethylendiamin mit einem 6-Aminocapronitril-Gehalt von 115 ppm abgezogen. Über Sumpf der Kolonne K1 wurden 156.5 kg/h mit einem Hexamethylendiamin-Gehalt von 0.05 % abgezogen. Um diesen Hexamethylendiamin-Gehalt im Sumpf der Kolonne zu erhalten, waren 140 kg/h Dampf nötig. In einer zweiten Destillationskolonne K2 wurde der Sumpfabzug aus Kolonne K1 so destilliert, dass 116.5 kg/h 6-Aminocapronitril über Kopf abgezogen wurden. Der Hexamethylendiamin-Gehalt im Kopfabzug betrug 800 ppm. Die Kolonne wurde mit 30 kg/h Dampf betrieben.

Trotz des höheren Energieverbrauches der Kolonne K1 mit 140 kg/h im Vergleichsbeispiel als die Summe der Energieverbräuche in Kolonnen K1 und K3 gemäß Beispiel mit insgesamt 95 kg/h wurde im Vergleichsbeispiel eine deutlich schlechtere ACN-Reinheit erreicht.

## Patentansprüche

1. Verfahren zur destillativen Abtrennung von 6-Aminocapronitril aus Gemischen, die 6-Aminocapronitril, Adipodinitril und Hexamethylendiamin enthalten, wobei man
a) Hexamethylendiamin aus dem Gemisch abtrennt, unter Erhalt einer Mischung (I), die einen Hexamethylendiamin-Gehalt von weniger als 1 Gew.-% enthält
b) 6-Aminocapronitril ganz oder teilweise aus Mischung (I) abtrennt, unter Erhalt einer Mischung (II), deren Gehalt an Stoffen, die unter Destillationsbedingungen höher als 6-Aminocapronitril sieden und nicht durch Dimerisierungsreaktionen bei thermischer Belastung von 6-Aminocapronitril entstehen können, kleiner als 1 Gew.-% ist und
c) aus Mischung (II) ganz oder teilweise vorhandenes Hexamethylendiamin abtrennt unter Erhalt einer Mischung (IV), deren Hexamethylendiamin-Gehalt höher als in Mischung (II) ist und einer Mischung (V), deren Hexamethylendiamin-Gehalt geringer als in Mischung (II) ist.

2. Verfahren nach Anspruch 1, wobei man in Schritt a Hexamethylendiamin an einem Seitenabzug gewinnt.

3. Verfahren nach Anspruch 1 oder 2, wobei man Hexamethylendiamin und bezüglich Hexamethylendiamin leichter siedende Komponenten gemeinsam abtrennt.

4. Verfahren nach den Ansprüchen 1 bis 3, wobei man die in Schritt c abgetrennte Mischung (IV) teilweise oder vollständig in Schritt a zurückführt.

5. Verfahren nach Anspruch 4, wobei die aus Schritt c in Schritt a zurückgeführte Mischung (IV) dampfförmig ist.

6. Verfahren nach Anspruch 5, wobei man Mischung (VII) teilweise oder vollständig in Schritt b zurückführt.

7. Verfahren nach den Ansprüchen 1 bis 6, wobei der Druck in Schritt b so gewählt wird, daß die Sumpftemperatur 185°C nicht übersteigt.

## Claims

1. A process for the distillative separation of 6-aminocapronitrile from mixtures comprising 6-aminocapronitrile, adipodinitrile and hexamethylenediamine, wherein
a) the hexamethylenediamine is separated from the mixture to give a mixture (I) which comprises a hexamethylenediamine content of less than 1% by weight;
b) all or part of the 6-aminocapronitrile is separated from the mixture (I) to give a mixture (II) whose content of substances which boil above 6-aminocapronitrile under distillation conditions and cannot be formed by dimerization reactions of 6-aminocapronitrile under thermal stress is less than 1% by weight; and
c) all or part of the hexamethylenediamine present is separated from the mixture (II) to give a mixture (IV) whose hexamethylenediamine content is higher than that of the mixture (II), and a mixture (V) whose hexamethylenediamine content is lower than that of the mixture (II).

2. The process according to claim 1 wherein hexamethylenediamine is recovered at a side discharge in step a.

3. The process according to claim 1 or 2 wherein hexamethylenediamine and components boiling below hexamethylenediamine are separated off together.

4. The process according to any of claims 1 to 3 wherein all or part of the mixture (IV) separated off in step c is recycled into step a.

5. The process according to claim 4 wherein the mixture (IV) recycled from step c into step a is vaporous.

6. The process according to claim 5 wherein all or part of the mixture (VII) is recycled into step b.

7. The process according to any of claims 1 to 6 wherein the pressure in step b is chosen so that the bottom temperature does not exceed 185°C.

## Revendications

1. Procédé de séparation de 6-aminocapronitrile par distillation de mélanges qui contiennent du 6-aminocapronitrile, de l'adipodinitrile et de l'hexaméthylènediamine, dans lequel
a) on sépare l'hexaméthylènediamine à partir du mélange, avec obtention d'un mélange (I), qui contient une teneur en hexaméthylènediamine inférieure à 1 % en poids,
b) on sépare le 6-aminocapronitrile totalement ou partiellement à partir du mélange (I), avec obtention d'un mélange (II) dont la teneur en substances, qui, dans des conditions de distillation, ont un point d'ébullition plus élevé que le 6-aminocapronitrile et qui ne peuvent pas se former par des réactions de dimérisation lors d'une sollicitation thermique du 6-aminocapronitrile, est inférieure à 1 % en poids, et
c) on sépare le mélange (II) totalement ou partiellement de l'hexaméthylènediamine présente, avec obtention d'un mélange (IV), dont la teneur en hexaméthylènediamine est plus élevée que dans le mélange (II) et d'un mélange (V), dont la teneur en hexaméthylènediamine est plus faible que dans le mélange (II).

2. Procédé suivant la revendication 1, dans lequel, dans l'étape a, on obtient l'hexaméthylènediamine à un soutirage latéral.

3. Procédé suivant la revendication 1 ou 2, dans lequel on isole conjointement de l'hexaméthylènediamine et des composants à point d'ébullition plus bas que l'hexaméthylènediamine.

4. Procédé suivant les revendications 1 à 3, dans lequel on recycle partiellement ou totalement dans l'étape a le mélange (IV) isolé dans l'étape c.

5. Procédé suivant la revendication 4, dans lequel le mélange (IV) recyclé de l'étape c à l'étape a est sous forme de vapeur.

6. Procédé suivant la revendication 5, dans lequel on recycle partiellement ou totalement dans l'étape b le mélange (VII).

7. Procédé suivant les revendications 1 à 6, dans lequel la pression dans l'étape b est choisie de façon que la température de cuve ne dépasse pas 185°C.
